# EUROPEAN PATENT APPLICATION

(11) **EP 2 769 974 A1**
(43) Date of publication of application: **27.08.2014**
(21) Application number: 13000883.2
(22) Date of filing: 21.02.2013
(51) Int. Cl.: C07D 209/42, A61K 31/404, A61P 3/10, A61P 3/04

(54) **Novel AMPK activator**

(71) Applicant: Debiopharm S.A., 1002 Lausanne (CH)
(72) Inventor: The designation of the inventor has not yet been filed

(57) **Abstract**

The present invention relates to an indole derivative of structural formula (I), or a pharmaceutically acceptable salt or solvate thereof, which is useful as a direct AMPK activator, to uses and methods of use thereof, and to pharmaceutical compositions comprising such compound.

## Description

### FIELD OF THE INVENTION

The present invention relates to a compound of structural formula (I), to medicaments and pharmaceutical compositions comprising such compound, as well as to uses and methods of use of such compound in the treatment of disorders related to AMPK regulation.

### BACKGROUND

Metabolic disorders such as diabetes, dyslipidemia, hypertension, overweight, obesity, etc. are well recognized as factors increasing the cardiovascular risk.

For example, diabetes, and particularly type 2 diabetes, is a complex metabolic disorder with many risk factors and implications. In patients with type 2 diabetes, hyperglycemia is the major risk factor for microvascular complications, and 70% to 80% of such patients will die of macrovascular disease. Type 2 diabetes patients with atherogenic dyslipidemia, which includes elevated plasma triglycerides, low HDL-C and a preponderance of small dense LDL particles, have a higher risk of atherosclerosis. Therefore, treatment of type 2 diabetes should address both hyperglycemia to prevent microvascular disease (such as retinopathy, neuropathy, nephropathy) and atherogenic dyslipidemia to prevent macrovascular complications (Reasner CA. J Cardiovasc Pharmacol, 2008, 52(2):136-44). This currently requires multiple medications, including antidiabetic and lipid-lowering agents (and antihypertensive agents, if necessary), to sufficiently manage all aspects of the pathology of this disease. Unfortunately, only about 7% of patients with diabetes achieve control of their hyperglycemia (A1C <7.0%), dyslipidemia (total cholesterol <200 mg/dL [5.18 mmol/L]), and hypertension (blood pressure <130/80 mm Hg) (Saydah SH et al. JAMA, 2004; 291:335-342).

The AMP-activated protein kinase (AMPK) acts as an intracellular metabolic sensor in a variety of cells, where it monitors and responds to variations in the AMP;ATP ratio (Hardie et al, Annu. Rev. Biochem. 67:821-855, 1998). AMPK is switched on by any cellular stress that causes a rise in the AMP:ATP ratio, either by interfering with ATP production (e.g. hypoxia, glucose deprivation or ischemia), or increasing ATP consumption (e.g. muscle contraction) (Hardie DG, Ross FA, Hawley SA., Nat Rev Mol Cell Biol. 13(4):251-62, 2012). Upon activation of AMPK, the enzyme phosphorylates a number of protein substrates to decrease further ATP usage by the cell.

Besides its function as a sensor of cellular energy status, AMPK also plays a critical role in the energy balance of the whole organism. For example, activation of AMPK switches on catabolic pathways that generate ATP in skeletal muscle and in the heart (glucose uptake and glycolysis, fatty acid uptake and oxidation), while it switches off ATP consuming processes, in particular anabolic (biosynthetic) pathways (fatty acid synthesis in adipose cells, fatty acid and cholesterol synthesis as well as gluconeogenesis in liver) (Hardie DG, Ross FA, Hawley SA., Nat Rev Mol Cell Biol. 2012 Mar 22;13(4):251-62). In addition, AMPK is activated, for example, by hormones and cytokines, including leptin and adiponectin, which increase AMPK signalling.

All those properties combine to make activation of AMPK a target of choice in the treatment of metabolic disorders.

AMPK activity is found in all tissues, including liver, kidney, muscle, lung, and brain (Cheung et al., Biochem, J. 346:659-669, 2000). In terms of structure, AMPK is a heterotrimeric complex consisting of a catalytic subunit (α) and two regulatory subunits (β and γ). The AMPK complex is evolutionarily conserved and also can be found in yeast and plants. Mammalian AMPK is composed of different isoforms of subunits: α1, α2, β1, β2, γ1, γ2, and γ3 (Hardie and Hawley, BioEssays 23:1112-1119, 2001) leading to 12 possible heterotrimeric combinations. The α2 isoform is predominately found in skeletal and cardiac muscle AMPK; both the α1 and α2 isoforms are found in hepatic AMPK; while in pancreatic islet β-cells the α1 isoform AMPK predominates (Quentin T et al., Histol Histopathol. 2011, 26(5);589-96).

It has been shown that metformin activates AMPK by an indirect mechanism, i.e. inhibition of complex I of the respiratory chain (Owen MR et al, Biochemical Journal, 2000, 348, 607-614), suggesting that it may activate AMPK by increasing cellular AMP:ATP ratio. Thiazolidinediones (TZD) can also activate AMPK by an indirect effect via the adiponectin release (Hardie DG. Annual Review of Pharmacology and Toxicology, 2007, 47;185-210), which may account for many of the long term effects of TZD, and by an adiponectin-independent effect, probably the inhibition of complex I of the respiratory chain, and thus increase of cellular AMP;ATP ratio, as metformin does.

There is currently hardly any drug available that is active on more than one factors of the cardiovascular risk. Thus, in view of minimizing the need for multiple medications to treat a single patient, there remains a need for a drug that acts on more than one factors of the cardiovascular risk in patients with metabolic disorders. The present invention aims at addressing such need.

### SUMMARY OF THE INVENTION

The present invention relates to a compound of structural formula (I) 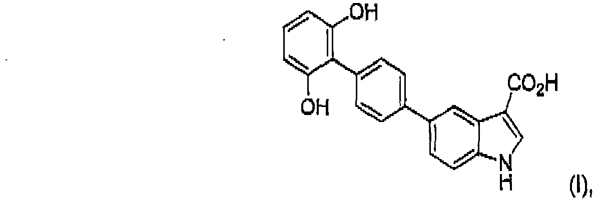 or a pharmaceutically acceptable salt or solvate thereof.

The present invention further relates to the compound of formula (I) as a medicament, to a medicament comprising compound of formula (I), or to the use of compound of formula (I) as a medicament. In particular, the present invention relates to the compound of formula (I) for use as a direct AMPK activator.

The present invention further relates to the compound of formula (I) for use in, or for use in the preparation of a medicament for, treating or preventing a disorder responsive to AMPK activation, as well as to a method comprising administering a therapeutically effective amount of the compound of formula (I) for treating or preventing a disorder responsive to AMPK activation.

In a particular embodiment, the disorder responsive to AMPK activation is a metabolic disorder. In further particular embodiments, the metabolic disorder is diabetes or dyslipidemia.

The present invention further relates to a pharmaceutical composition comprising a therapeutically effective amount of the compound for formula (I), or a pharmaceutically acceptable salt or solvate thereof, as active ingredient, and a pharmaceutically acceptable carrier. In a particular embodiment, the pharmaceutical composition is an oral dosage form,

### BRIEF DESCRIPTION OF THE DRAWINGS

Figure 1 is a diagram showing High Fat Diet (HFD) mice fasting plasma glycemia as obtained in the experiment described in Example 3a [*** p<0,001 Control Chow Diet (CD) vs Control HFD, *p < 0.05 Compound of formula (I) vs Control HFD].
Figure 2 is a diagram showing the HFD mice liver glucose production as obtained in the experiment described in Example 3b [**p < 0.005 Control CD vs Control HFD, ***p < 0.001 Compound of formula (I) vs Control HFD].
Figure 3 is a diagram showing HFD mice liver lipid levels as obtained in the experiment described in Example 4 [*** p<0.001 Control CD vs Control HFD. **p < 0.005 Compound of formula (I) vs Control HFD].
Figure 4 is a diagram showing the HFD hamster lipoprotein profiles as obtained in the experiment described in Example 5.

### DETAILED DESCRIPTION OF THE INVENTION

The present invention relates to a compound of structural formula (I) 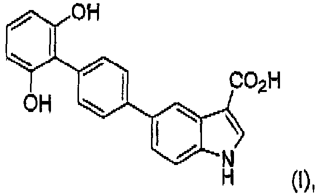 or a pharmaceutically acceptable salt or solvate thereof, which is useful as a direct AMPK activator, to uses and methods of use thereof, and to pharmaceutical compositions comprising such compound.

### Definitions

So that the invention may be more readily understood, certain terms are specifically defined below. Unless explicitly defined elsewhere in this document, all other technical and scientific terms used herein have the meaning that would be commonly understood by one of ordinary skill in the relevant art.

As used herein, including in the appended claims, the singular forms of words such as "a", "an", and "the", include their corresponding plural references unless the context clearly indicated otherwise.

As used herein, an "AMPK activator" refers to a compound that either increases the phosphorylation of downstream substrates of (phosphorylated or not) AMPK, and/or that increases the phosphorylation of AMPK.

As used herein, a "direct AMPK activator" refers to a compound that activates AMPK via direct interaction with at least one of its subunits.

As used herein, a "disorder responsive to AMPK activation" refers to a disorder, the symptoms of which would be alleviated, or the course of which would be beneficially modified, through activation of AMPK, including without limitation, metabolic disorder, diabetes, dyslipidemia, hypertension, overweight, obesity.

As used herein, the term "diabetes" includes insulin-dependent diabetes mellitus (i.e., IDDM, also known as type 1 diabetes) non-insulin-dependent diabetes mellitus (i.e., NIDDM, also known as type 2 diabetes), and prediabetes. Type 1 diabetes is the result of an absolute deficiency of insulin, the hormone which regulates glucose utilization. Type 2 diabetes often occurs in the face of normal, or even elevated levels of insulin and appears to be the result of the inability of tissues to respond appropriately to insulin. Most of the type 2 diabetic patients are also overweighed or obese. One of the criteria for diagnosing diabetes is the fasting plasma glucose level, A diabetic subject has a fasting plasma glucose level of greater than or equal to 126 mg/dl. A pre-diabetic subject is someone suffering from prediabetes, Prediabetes is characterized by an impaired fasting plasma glucose level of greater than or equal to 100 mg/dl and less than 126 mg/dl; or impaired glucose tolerance; or insulin resistance. A prediabetic subject is a subject with impaired fasting glucose (a fasting plasma glucose level of greater than or equal to 100 mg/dl and less than 126 mg/dl); or impaired glucose tolerance (a 2-hour plasma glucose level of ≥140 mg/dl and <200 mg/dl); or insulin resistance, resulting in an increased risk of developing diabetes. Prevention of type 2 diabetes includes treatment of prediabetes,

As used herein, the term "dyslipidemia" encompasses abnormal levels of any lipid fractions as well as specific lipoprotein abnormalities. For example, it refers to elevation of plasma cholesterol and/or elevation of triglycerides and/or elevation of free fatty acids and/or low high-density lipoprotein (HDL) level and/or high low-density lipoprotein (LDL) level and/or high very low-density lipoprotein (VLDL) level. Dyslipidemia may for example contribute to the development of atherosclerosis and ultimately symptomatic vascular disease including coronary heart disease. Dyslipidemia may or may not be associated with diabetes.

As used herein, the term "metabolic disorder" encompasses any abnormal chemical reaction disrupting normal metabolism, leading to excessive levels or deficiency of certain substances. Non-limiting examples of metabolic disorders include diabetes, dyslipidemia, hypertension, overweight, obesity, and any combination thereof.

A "patient" refers to a mammal, preferably a human.

As used herein, the expression "oral dosage form" refers to any form of a pharmaceutical composition that is suitable for oral administration.

As used herein, the expression "pharmaceutically acceptable salt" refers to salts prepared from pharmaceutically acceptable non-toxic bases or acids including inorganic or organic bases and inorganic or organic acids. Salts derived from inorganic bases include aluminium, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethano, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. The term "pharmaceutically acceptable salt" further includes all acceptable salts derived from acids such as quaternary salt, acetate, carbonate, carbamate, sulfonate, strong inorganic acids and the like. In general, pharmaceutically acceptable salts may be used for modifying the solubility or hydrolysis characteristics of a compound, or in sustained release formulations. It will be understood that, as used herein, references to the compound of formula (I) are meant to also include the pharmaceutically acceptable salts.

As used herein, the term "preventing" (or "prevent" or "prevention") means prohibiting, restraining, or inhibiting the incidence, occurrence or recurrence of a symptom, disorder, condition, or disease. For example, prevention of diabetes, such as diabetes associated with obesity, refers to the administration of a compound of the present invention to prevent the onset of diabetes in a subject in need thereof. A subject in need of preventing diabetes is, for example, a prediabetic subject that is overweight or obese.

As used herein, the expression "therapeutically effective amount" means the amount of the compound of formula (I) that will elicit the biological or medical response of a tissue, system, animal or human that is being sought by the researcher, veterinarian, medical doctor or other clinician, Such response includes, in comparison with a non-treated tissue, system, animal or human; alleviation of the symptoms of the disorder being treated; improved treatment; prevention, elimination or reduction of progress of a disease, condition or disorder. The expression "therapeutically effective amount" also encompasses the amounts which are effective for increasing normal physiological function, upon single or multiple administration of the compound. The therapeutically effective amount can, for example, comprise an amount of about 0.1 to 30 mg/kg per single administration.

As used herein, the term "treating" (or "treat" or "treatment") means slowing, stopping, reducing, alleviating or reversing the progression or severity of a symptom, disorder, condition or disease.

### Compound of formula (I) and uses thereof

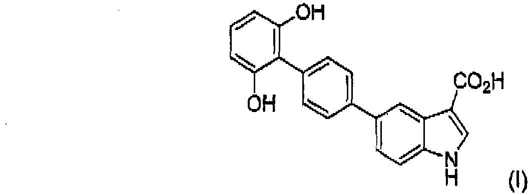

The compound of the present invention may be in the form of a solid (such as a beige solid as mentioned in Example 1 below, or a light brown to white solid), or in the form of a pharmaceutically acceptable salt. In addition, the compound of the instant invention may form solvates with water or common organic solvents (non-limiting examples of which are esters, alcohol, ethers, ketones, acid or DMSO). Such solvates are encompassed within the scope of this invention.

The compound of formula (I) is an activator of at least four heterotrimeric isoforms of human recombinant AMPK. It has been shown to have an EC50 of less than 0.5 µM on at least two β1-containing heterotrimers and less than 5 µM on at least two β2-containing heterotrimers, e.g. in the method described at Example 2.

It has been surprisingly found that the compound of formula (I) acts on both the glucose metabolism and the lipid metabolism *in vivo*, which makes it a drug of choice for treatment or prevention of metabolic disorders, including diabetes and dyslipidemia.

In particular, it has been observed, in a model of high fat diet (HFD) mice, that compound of formula (I) orally administered at 60 mg/kg twice a day decreases hepatic glucose production by at least 34% in basal state, decreases fasting plasma glycemia by at least 17% (see e,g. Example 3).

The compound of formula (I) also showed a strong decrease in all lipid fraction levels in the liver of HFD mice (by at least 40% to 70% for cholesterol, triglycerides, fatty acids), when orally administered at 60 mg/kg (see e.g. Example 4).

In addition, an oral dose of 60 mg/kg of the compound of formula (I) markedly improved dyslipidemia in a hamster model by increasing HDL (by at least 40% to 70%), decreasing non-HDL, in particular VLDL (by at least 34% to 74%) and showing a decrease in LDL (by at least 11 % to 49%), in the circulating lipids of HFD hamsters (see e.g. Example 5).

The compound of formula (I) also improved insulin sensitivity (HOMA-IR decreased by at least 37% to 43%) in HFD mouse and HFD hamster models.

Thus the compound of formula (I) presents advantageous properties in decreasing fasting blood glucose, decreasing all fractions of liver lipids and improving circulating lipid profile. These properties can be beneficially used for treating or preventing a variety of disorders related to glucose and/or lipid metabolism in patients in need thereof. Compound of formula (I) is thus particularly advantageous as none of the currently available drugs presents all of these effects combined, In addition, the combined effects of the compound of structural formula (I) could not be predicted from previously known structures.

Accordingly, the present invention further relates to uses of the compound of formula (I) in the treatment or prevention of disorders responsive to AMPK activation, such as metabolic disorders. In a particular embodiment thereof, the present invention relates to the use or method of use of the compound of formula (I) in the treatment or prevention of diabetes. In another particular embodiment, the present invention relates to the use or method of use of the compound of the present invention in the treatment or prevention of dyslipidemia. It is to be understood that prevention or treatment of several of these disorders may be combined while using the compound of formula (I),

### Pharmaceutical compositions

In another aspect, the present invention further relates to pharmaceutical compositions comprising a therapeutically effective amount of the compound of formula (I), or a pharmaceutically acceptable salt or solvate thereof, as active ingredient, and a pharmaceutically acceptable carrier. For example, it relates to pharmaceutical compositions comprising a therapeutically effective amount of an ammonium salt, a calcium salt, a magnesium salt, a potassium salt or a sodium salt of compound of formula (I) and a pharmaceutically acceptable carrier.

The term "composition", as in pharmaceutical composition, is intended to encompass a product comprising the active ingredient, and the inert ingredient(s) (pharmaceutically acceptable excipients) that make up the pharmaceutically acceptable carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing the compound of formula (I) and pharmaceutically acceptable excipients.

Any suitable route of administration may be employed for providing a patient, with an effective dosage of a compound of the present invention, including without limitation oral and parenteral (such as intravenous bolus or infusion, injection, intraperitoneal, subcutaneous or intramuscular administration).

Pharmaceutical compositions of the present invention that are suitable for oral administration (oral dosage forms) may be presented in solid or liquid form. Suitable solid oral dosage forms include discrete units such as capsules, pills, cachets, powders (such as effervescent), granules or tablets, and the like, each containing a predetermined amount of the compound of formula (I) as active ingredient. Suitable liquid oral dosage forms include solutions or suspensions in an aqueous liquid, a non-aqueous liquid, an oil-in-water emulsion or a water-in-oil liquid emulsion, including elixirs, tinctures, solutions, suspensions, syrups and emulsions. Pharmaceutical compositions of the present invention may also be in the form of sustained release formulations.

Any inert ingredient that is commonly used as a carrier or diluent may be used in the formulations of the present invention, such as for example, a gum, a starch, a sugar, a cellulosic material, an acrylate, or mixtures thereof. A preferred diluent is microcrystalline cellulose. The compositions may further comprise a disintegrating agent (e.g., croscarmellose sodium) and a lubricant (e.g., magnesium stearate), and may additionally comprise one or more additives selected from a binder, a buffer, a protease inhibitor, a surfactant, a solubilizing agent, a plasticizer, an emulsifier, a stabilizing agent, a viscosity increasing agent, a sweetener, a film forming agent, or any combination thereof.

The pharmaceutical compositions of the present invention may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent, Desirably, each tablet, cachet or capsule contains from about 0.1 to 1,000 mg, particularly 0.1, 0.2, 0.5,1,0, 5, 10, 25, 50, 75, 100, 110, 115, 120, 125, 130, 135, 140, 145, 150, 175, 180, 200, 225, 250, 300, 350, 400, 450, 500, 750 and 1,000 milligrams of the active ingredient, for the symptomatic adjustment of the dosage to the patient to be treated.

### Method of preparation of compound of formula (I)

The compound of structural formula I of the present invention can be prepared, for example, according to the procedures presented below. In particular, Example 1 illustrates details for the preparation of the compound of the present invention. Those skilled in the art will readily understand that known variations of the conditions and processes of the following preparative procedures can be used to prepare these compounds. The instant compound is generally isolated in the form of a pharmaceutically acceptable salt, such as those previously described herein, or of a solid. The use of protecting groups for the alcohol functionalities to facilitate the desired reaction and minimize undesired reactions is well documented. Conditions required to remove protecting groups are found in standard textbooks such as Greene, T, and Wuts, P. G. M., Greene's Protective Groups in Organic Synthesis, John Wiley & Sons, Inc., 2007, 4th ed, New Jersey. For example, OMe functionalities are commonly used protecting groups in organic synthesis, and their removal conditions are known to those skilled in the art. All temperatures are degrees Celsius unless otherwise noted. Mass spectra (MS) were measured by electron-spray ion-mass spectroscopy.

### EXAMPLES

### EXAMPLE 1: synthesis scheme of compound of formula (I)

Abbreviations used in the present example are as follows: BBr₃ is boron tribromide; Cl₃CCOCl is trichloroacetyl chloride; Cs₂CO₃ is cesium carbonate; DCM is dichloromethane; DME is 1,2-dimethoxyethane; EtOAc is ethyl acetate; HCl is hydrochloric acid; K₂CO₃ is potassium carbonate; KOH is potassium hydroxide; MeOH is methanol; NaCl is sodium chloride; Na₂SO₄ is sodium sulfate; Pd(PPh₃)4 is Tetrakis(triphenylphosphine)palladium(0).

### Stage 1: Preparation of 5-(4-Bromophenyl)-1H-indole (2)

To a solution of indole-5-boronic acid **(1)** (14.2 g, 88.37 mmol) in 1,4-dioxane:water (4;1 ^{V}/_{V,} 398 mL) (1 L flask) was added 4-iodo-1-bromobenzene (25.0 g, 88,37 mmol), potassium carbonate (24,8 g, 176.74 mmol) and Pd(PPh₃)₄ (5.11 g, 4,42 mmol) under inert atmosphere. The reaction mixture was heated at 90°C for 22 hours. After completion, the resulting mixture was cooled to room temperature and filtered through pad of celite to remove inorganic salts insoluble in the mixture. The organic phase was concentrated *in vacuum* to give the crude as orange-brown oil. The reaction mixture was diluted with DCM (600 mL) and washed with sat. aq. NaCl (500 mL), water phase was re-extracted with DCM (200 mL) and the combined organic phases were dried over Na₂SO₄, filtered and evaporated under reduced pressure. The material was further purified by column chromatography (gradient EtOAc in Hexane). The desired product **(2)** (20.8 g, 87%) was obtained as an off-white solid.

### Stage 2: Preparation of 1-[5-(4-Bromophenyl)-1H-indole-3-yl]-2,2,2-trichloroethane-1-one (3)

Using a 250-mL one-necked, round-bottomed flask equipped with a magnetic stirring bar, a solution of 5-(4-bromophenyl)-1*H*-indole (5.0 g, 18.4 mmol) in 1.4-dioxane (150 mL, 30 vol.) was prepared. To this pale yellow mixture, pyridine (14.8 mL, 180.4 mmol,) was added slowly at room temperature under nitrogen atmosphere. The mixture was cooled with an ice bath (10-15 °C) and the trichloroacetyl chloride (10.3 mL, 92.0 mmol, 5.0 eq.) was added dropwise; at the end of addition the reaction mixture was heated at 80 °C for 4 hrs 30 min. The mixture was cooled to room temperature and EtOAc (700 mL) and 1 M HCl (300 mL) were added and the mixture was shaken vigorously. An aqueous layer was re-extracted with EtOAc (100 mL). Both organic extracts were combined, washed with brine (100 mL), separated, dried over Na₂SO₄, filtered and evaporated under reduced pressure. The crude product was suspended in EtOAc (100 mL) and hexane (100 mL). The resulting suspension was stirred for 10 min, filtered off, the solid was washed with cold EtOAc (20 mL) and dried under vacuum to give the desired product **(3)** (7.05 g, 91.8%) as a beige solid.

### Stage 3: Preparation of methyl 5-(4-bromophenyl)-1H-indole-3-carboxylic-acid (4)

To a suspension of **(3)** (7.05 g, 16.88 mmol, 1.0 equiv,) in dry MeOH (350 mL), aq. 50% KOH by wt. (∼ 0.6 mL) was added dropwise to pH = 9-10. The resulting beige suspension was vigorously stirred under reflux for 4 h. Subsequently, the reaction mixture with solid present was cooled down to room temperature and concentrated under reduced pressure. The desired product was collected by filtration, washed with water (2 x 20 mL) and dried under high vacuum overnight to give **(4)** as a beige-brown powder (5.40 g, 96.9%).

### Stage 4: Preparation of methyl 5-(2',6'-dimethoxy-biphenyl-4-yl)-1H-indole-3-carboxylic acid (5)

To a solution of methyl 5-(4-bromophenyl)-1*H*-indole-3-carboxylate **(4)** (1.0 g, 3,02 mmol,1.0 equiv.) in DME/water (6:1, 20 mL) (100 mL flask), 2,6-dimethoxyphenylboronic acid (0.55 g, 3.02 mmol, 1.0 equiv.), cesium carbonate (1.97 g, 6.04 mmol, 2.0 equiv,) and Pd(PPh₃)₄ (0.35g, 0.302 mmol, 10 mol %) were added under inert atmosphere. The reaction mixture was heated under reflux for 22 hours. The mixture was cooled to room temperature, filtered through a pad of celite (2 g), the pad of celite was washed with DCM (2 times 10 mL) and the mixture was concentrated *in vacuo* to give the crude product, Subsequently, the crude material was dissolved in DCM (100 mL), the organic layer was washed with 10% citric acid (50 mL), aq. phase was re-extracted with DCM (3 x 100 mL). The combined organic phases were washed with brine (50 mL) and dried over Na₂SO₄. The solvent was evaporated under reduced pressure. Hexane was added (approx. 100 mL) to the precipitate and the product was filtered off to afford 1.04 g (92%) of **(5)** as a yellowish powder.

### Stage 5: Preparation of 5-(2',6'-dihydroxy-biphenyl-4-yl)-1H-indole-3-carboxylic acid (6) (compound of formula (I))

To a suspension of methyl 5-(2',6'-dimethoxy-biphenyl-4-yl)-1*H*-indole-3-carboxylate **(5)** (0.8 g, 2.06 mmol) in DCM (50 mL) was added BBr₃ (6.19 mL, 6.19 mmol, 3.0 equiv.) at -50 °C as 1 M DCM solution and stirred to 0 °C for 1 h and then overnight (21 hrs) at rt. The reaction mixture was quenched with H₂O (10 mL) and aq. sat. NaHCO₃ (pH = 9) followed by dilution with DCM (50 mL), to this water (20 mL) and brine (20 mL) wash was given, the organic layer was separated, dried over Na₂SO₄ and concentrated to dryness under reduced pressure. The crude was purified by column chromatography on silica gel, (gradient EtOAc in Hexane) to give the desired product **(6)** as a beige solid (0.33 g, 45%).

### EXAMPLE 2: in vitro activity of compound of formula (I)

The compound of formula (I) was assayed in vitro to determine its EC50 against four different AMPK heterotrimers (two containing the β1 subunit and two containing the β2 subunit). AMPK activity was measured by the phosphorylation of the amino-terminal fragment of human acetyl CoA carboxylase-type 1, amino acids 1-120 (ACC1/1-120), at ser79. The fragment was expressed as a biotinylated fusion protein in E.coli. Human rAMPK genes were expressed in insect cells using baculovirus vectors or in the monkey COS7 cell expression systems. The compound was tested in triplicate at each of the following concentrations (µM): 45, 15, 5, 1.67, 0.56, 0.185, 0.06, 0.02 and 0.00685. A positive control comprising AMP (100 uM), and a negative control without enzyme, were used. The enzyme assay was conducted in a 5 µl reaction mixture containing compound of formula (I) at the indicated concentrations in 80 mM HEPES, pH7.0, 160 mM NaCl, 2.5 mM MgCl₂, 1mM DTT, 0.05% Tween-20, 100 µM ATP with AMPK at 0.08 ng/µl and ACC1/1-120 at 0.004 ng/µl. The reaction was carried out at room temperature for 60 minutes and stopped by addition of 5 µl of stop solution consisting of 20 mM EDTA, 100 mM Tris pH8,0, 0.01 % Tween-20, 0.1% BSA, 1:2,000 dilution of anti-pS79 (Acetyl CoA carboxylase-1) purified rabbit monoclonal antibody, 40 µg/ml of AlphaScreen (Perkin Elmer) acceptor beads and 40 µg/ml of AlphaScreen (Perkin EImer) donor beads. The reaction mixture was further incubated overnight at RT and analyzed on a Perkin EImer Fusion-Alpha microplate analyzer. The AlphaScreen readout for this assay is based on an excitation with laser-stimulated visible light at 680 nm and emission at 520-620 nm.

EC50 was found to be less than 0.5 µM on the two β1-containing heterotrimers and less than 5 µM on the two β2-containing heterotrimers.

### EXAMPLE 3: in vivo activity of compound of formula (I) on glycemia

### Example 3a): Fasting plasma glycemia in high-fat diet-fed (HFD) mice

Six-week-old male C57BI/6 mice were fed a high-fat diet or chow diet for 3 months. Then, HFD mice were treated orally with vehicle (Control HFD) or compound of formula (I) at 60 mg/kg twice daily for 4 weeks. Chow fed mice were treated orally with vehicle alone (Control CD). At completion of this treatment period, mice were fasted for 6 hours and blood glucose levels were assessed with a blood glucosemeter from blood samples collected from the tip of the tail vein.

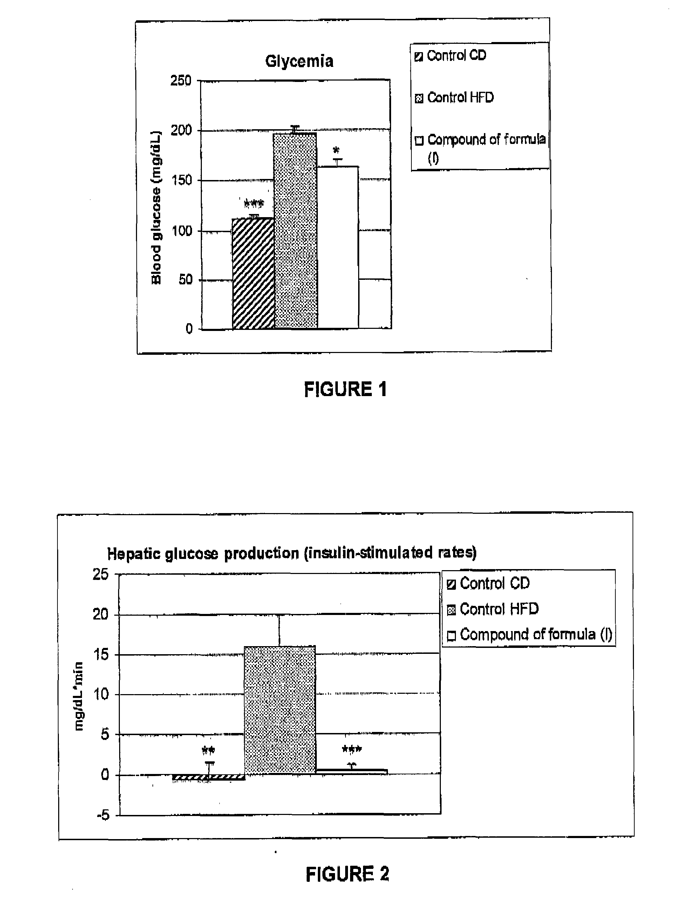

The results are shown on Figure 1. Blood glucose was increased in HFD mice versus chow fed mice (p<0.001). Compound (I) significantly decreased blood glucose of HFD mice (p <0.05). Results are presented as mean ± standard error (SEM) of 8-10 animals per group (*** p<0.001 Control CD vs Control HFD, *p < 0.05 Compound of formula (I) vs Control HFD). Thus compound of formula (I) improved fasting glucose in high-fat diet-fed (HFD) mice.

It also improved insulin sensitivity (HOMA-IR decreased by 43%) in this model (Results not shown).

### Example 3b); Hepatic glucose production in HFD mice

Six-week-old male C57BI/6 mice were fed a high-fat diet or chow diet for 3 months. Then, HFD mice were treated orally with vehicle (Control HFD) or compound of formula (I) at 60 mg/kg twice daily for 40 days. Chow fed mice were treated orally with vehicle alone (Control CD). Hepatic glucose production was assessed during a hyperinsulinemic-euglycemic clamp using a 12 mU/kg/min insulin infusion rate. Hyperinsulinemic-euglycemic clamps were performed in chronically cannulated awake free moving mice. Before the start of the clamp procedure mice were fasted for 6 hours. ³H-glucose alone was then infused for 210 minutes to assess the basal whole body glucose turnover (= hepatic glucose output). The basal procedure started with D-[3-3H]glucose, which was infused (2µL/min, 30µCi/min/kg) for 90 minutes (time -60 to time 30 minutes). An euglycemic hyperinsulinemic 1 step insulin clamp was then performed from 30 minutes to 150 minutes, with a 12mU/kg/min insulin infusion. Glucose (16.5%) infusion rate was adjusted until steady-state blood glucose (120 mg/dL, ± 10 mg/dL) was achieved. Calculations for glucose turnover measurements were made from parameters obtained during the last 60 min of the infusions in steady-state condition, Briefly, the D-[3-3H]glucose-specific activity was calculated by dividing the D-[3-3H]glucose enrichment by the plasma glucose concentration. The whole-body glucose turnover rate was calculated by dividing the rate of D-[3-3H]glucose by the D-[3-3H]glucose plasma-specific activity. For each mouse, the mean values were calculated and averaged with values from mice of the same group.

The results are shown on Figure 2. Hepatic glucose production was largely increased in the HFD mice versus chow fed animals. Compound of formula (I) treatment blunted hepatic glucose production under insulin stimulated states. Results are presented as a mean ± standard error (SEM) of 4-6 animals per group (**p < 0.005 Control CD vs Control HFD, ***p < 0.001 Compound of formula (I) vs Control HFD). Thus compound of formula (I) normalized hepatic glucose production in high-fat diet-fed (HFD) mice.

### EXAMPLE 4: in vivo activity of compound of formula (I) on hepatic lipids in HFD mice

Six-week-old male C57BI/6 mice were fed a high-fat diet or chow diet for 3 months. Then, HFD mice were treated orally with vehicle (Control HFD) or compound of formula (I) at 60 mg/Kg twice daily for 40 days. Chow fed mice were treated orally with vehicle alone (Control CD). At completion of this treatment period, mice were sacrificed and liver collected for determination of triglycerides, cholesterol and free fatty acids (FFA) content. Commercial kits were used to measure the levels of hepatic cholesterol (cholesterol RTU, Biomerieux), triglyceride (TG) (triglyceride PAP 150, Biomerieux), and fatty acid (NEFA-HR (2), Wako Chemicals) levels from liver homogenate. Results were then normalized to the weight of the sample.

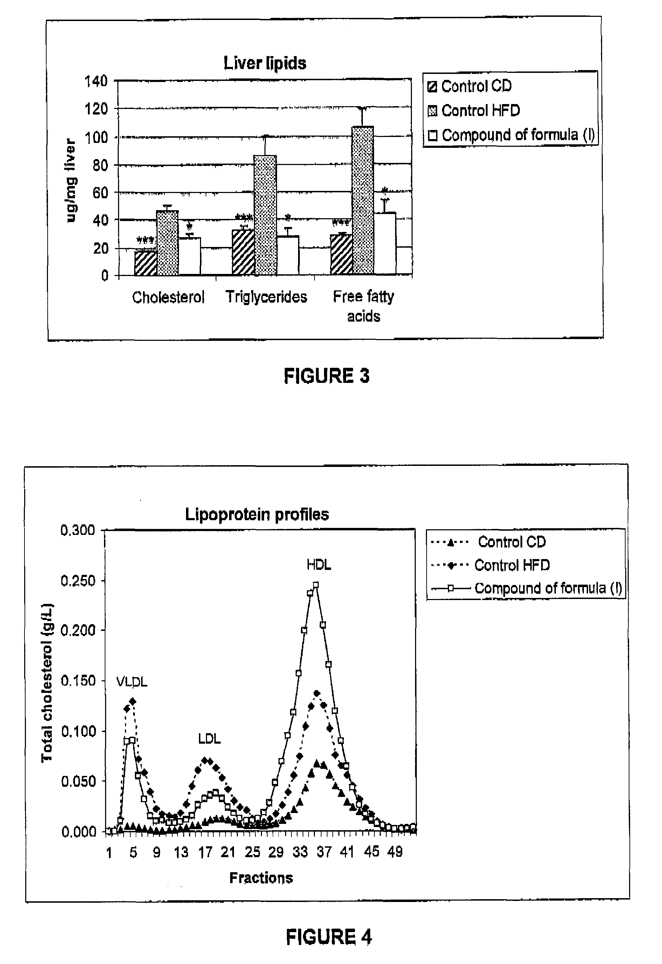

The results are shown on Figure 3. Liver lipid content was very high in HFD mice with large increase in liver cholesterol, FFA and triglyceride content. Compound of formula (I) reduced liver lipids content (p<0.001 vs control HFD) to near control chow values. Results are presented as a mean ± standard error (SEM) of 5-9 animals per group (*** p<0,001 Control CD vs Control HFD, **p < 0.005 Compound of formula (I) vs Control HFD). Thus compound of formula (I) corrected steatosis in high-fat diet-fed (HFD) mice.

### EXAMPLE 5: in vivo activity of compound of formula (I) on circulating lipids in HFD hamster

Hamsters were fed a control chow diet with normal drinking water or high fat diet with 10% fructose drinking water over 30 days. After 16 days of high-fat diet, two groups of hamsters were treated orally with either vehicle (control HFD) or Compound of formula (I) twice daily for 14 days. Hamsters fed the chow diet were also treated orally for 14 days with vehicle BID (control CD). After the treatment period, hamsters were overnight fasted, blood samples were obtained from the retroorbital plexus. Plasma was immediately isolated by centrifugation and for each group, a 400 µL pool of plasma was made from the individual plasma samples. Lipoproteins were separated by fast performance liquid chromatography (FPLC). FPLC fractions were monitored for cholesterol levels.

The VLDL, LDL, and HDL fractions after FPLC lipoprotein profiles analysis are shown in Figure 4. Compound of formula (I) reduced VLDL and LDL total cholesterol levels, while it increased HDL total cholesterol levels. Results are presented as means of 14 animals per group. Thus compound of formula (I) improved serum lipid profile in hamster on a high-fat diet.

The compound also improved insulin sensitivity (HOMA-IR decreased by 37%) in this model (Results not shown).

## Claims

1. A compound of structural formula (I) 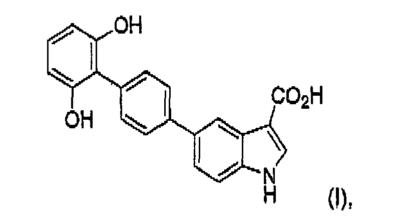 or a pharmaceutically acceptable salt or solvate thereof.

2. The compound as defined in claim 1, as a medicament.

3. The compound as defined in claim 1, for use as a direct AMPK activator.

4. The compound as defined in claim 1, for use in treating or preventing a disorder responsive to AMPK activation.

5. The compound as defined in claim 4, wherein the disorder responsive to AMPK activation is a metabolic disorder.

6. The compound as defined in claim 5, wherein the metabolic disorder is diabetes.

7. The compound as defined in claim 5, wherein the metabolic disorder is dyslipidemia.

8. A pharmaceutical composition comprising a therapeutically effective amount of the compound as defined in claim 1, or a pharmaceutically acceptable salt or solvate thereof, as active ingredient, and a pharmaceutically acceptable carrier.

9. The pharmaceutical composition as defined in claim 8, wherein said composition is an oral dosage form.
